(19) Europäisches Patentamt
European Patent Office
Office européen des brevets

(11) EP 3 061 742 A1

(12) EUROPEAN PATENT APPLICATION
published in accordance with Art. 153(4) EPC

(43) Date of publication:
**31.08.2016 Bulletin 2016/35**

(21) Application number: **14856110.3**

(22) Date of filing: **22.10.2014**

(51) Int Cl.:
*C07C 39/17* (2006.01) *B01J 27/053* (2006.01)
*C07C 37/16* (2006.01) *C08G 64/06* (2006.01)
*C07B 61/00* (2006.01)

(86) International application number:
**PCT/JP2014/078087**

(87) International publication number:
**WO 2015/060343 (30.04.2015 Gazette 2015/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.10.2013 JP 2013222022**

(71) Applicant: **Mitsubishi Gas Chemical Company, Inc.**
**Tokyo 100-8324 (JP)**

(72) Inventors:
- **NAKAMURA, Goh**
  **Kurashiki-shi**
  **Okayama 712-8525 (JP)**
- **OGURO, Dai**
  **Tokyo 100-8324 (JP)**
- **FUJITA, Hideaki**
  **Kurashiki-shi**
  **Okayama 712-8525 (JP)**
- **ZAIMA, Fumiya**
  **Tokyo 100-8324 (JP)**
- **SUGIYAMA, Genki**
  **Kamisu-shi**
  **Ibaraki 314-0102 (JP)**

(74) Representative: **Hoffmann Eitle**
**Patent- und Rechtsanwälte PartmbB**
**Arabellastraße 30**
**81925 München (DE)**

(54) **1,3-BIS(HYDROXYPHENYL)-5-ETHYLADAMANTANE COMPOUND AND METHOD FOR PRODUCTION THEREOF, AND AROMATIC POLYCARBONATE RESIN AND METHOD FOR PRODUCTION THEREOF**

(57) The present invention can provide a 1,3-bis(hydroxyphenyl)-5-ethyladamantane compound represented by Formula (1) below and a method for producing the same, as well as an aromatic polycarbonate resin comprising said compound and a method for producing the same.

[Chemical Formula 1]

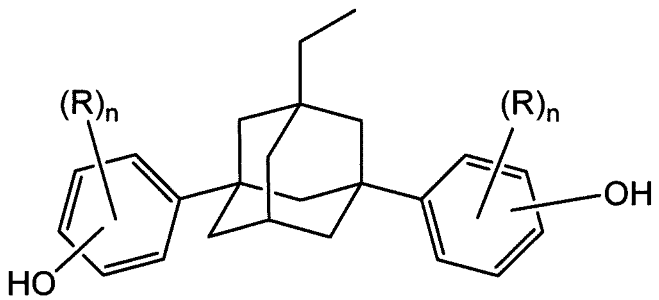

(1)

(wherein, R represents an alkyl group with a carbon number of 1-6, a cycloalkyl group with a carbon number of 3-6, or a phenyl group, and n represents an integer of 0-2).

EP 3 061 742 A1

## Description

TECHNICAL FIELD

The present invention relates to a novel 1,3-bis(hydroxyphenyl)-5-ethyladamantane compound which is a raw material beneficial in improving heat resistance, optical properties and mechanical strength properties in various resins and a method for producing the same, and an aromatic polycarbonate resin comprising said compound and a method for producing the same.

BACKGROUND ART

Resins that are produced using bisphenols as raw materials are used in various application by taking advantage of their heat resistance, optical properties and mechanical strength properties. Among the bisphenols used as the raw materials, a bisphenol having an adamantane backbone is described in Patent Document 1 as 1,3-bis(4-hydroxyphenyl)-5,7-dimethyladamantane synthesized by reacting 1,3-dibromo-5,7-dimethyladamantane with phenol.

In addition, Patent Document 2 describes 1,3-bis(4-hydroxyphenyl)adamantane synthesized by reacting 1,3-adamantanediol with phenol in the presence of an acid catalyst.

Furthermore, Patent Document 3 describes 1,3-bis(4-hydroxyphenyl)adamantanes and 1,3-bis(2-hydroxyphenyl)adamantanes that are synthesized by reacting 1,3-adamantanediols with a substituted phenol in the presence of an acid catalyst.

However, while only adamantane without a substituent and dimethyladamantane are conventionally known as adamantane backbone moieties of 1,3-bis(hydroxyphenyl)adamantanes, a 1,3-bis(hydroxyphenyl)-5-ethyladamantane compound that has ethyladamantane as an adamantane backbone moiety is unknown.

Moreover, Patent Document 4 describes, as a method for producing an ethyladamantane derivative, a method for producing 1,3-dihydroxy-5-ethyladamantane by oxidizing 1-ethyladamantane with chromic acid in an aqueous acetic acid solution.

Patent Document 5 describes an aromatic polycarbonate resin with superior heat resistance and optical properties that is produced by reacting an aromatic dihydroxy compound mainly composed of 1,3-bis(4-hydroxyphenyl)-5,7-dimethyladamantane with a polycarbonate precursor.

PRIOR ART DOCUMENTS

[Patent Documents]

Patent Document 1: US Patent No. 3,594,427
Patent Document 2: Japanese Patent Laid-open Publication No. 2000-95720
Patent Document 3: Japanese Patent Laid-open Publication No. 2003-306460
Patent Document 4: US Patent No. 3,383,424
Patent Document 5: Japanese Patent Laid-open Publication No. H05-78467

SUMMARY OF THE INVENTION

Problems to be Solved by the Invention

Although the aromatic polycarbonate resin described in Patent Document 5, which is obtained by using 1,3-bis(4-hydroxyphenyl)-5,7-dimethyladamantane as an aromatic dihydroxy compound, has very high heat resistance, molding is not easy because of its glass-transition temperature being too high and thus improvement has been required.

The problems of the present invention are to provide an aromatic polycarbonate resin that can realize both heat resistance and moldability, and to provide 1,3-bis(hydroxyphenyl)-5-ethyladamantane compound, i.e., a bisphenol compound having a novel adamantane backbone, as a raw material of said resin, and methods for producing the same.

Means for Solving the Problems

The present inventors have gone through keen examination, as a result of which found that 1,3-bis(hydroxyphenyl)-5-ethyladamantanes can be produced by reacting 1,3-dihydroxy-5-ethyladamantane with phenol or a substituted

phenol in the presence of an acid catalyst.

The present inventors have also found that an aromatic polycarbonate resin obtained by reacting said 1,3-bis(hydroxyphenyl)-5-ethyladamantanes with a carbonate-ester-forming compound is a resin that can realize both heat resistance and moldability.

The present invention was achieved based on these findings.

Thus, the present invention is as follows.

[1] A 1,3-bis(hydroxyphenyl)-5-ethyladamantane compound represented by Formula (1) below:

[Chemical Formula 1]

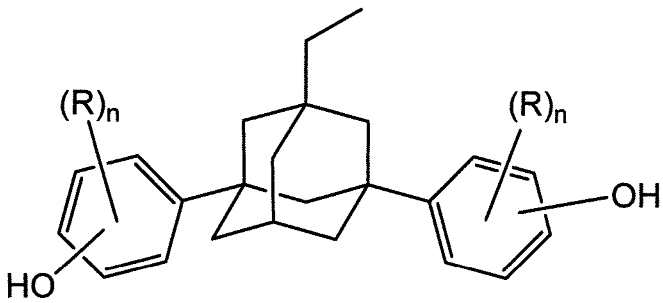

(1)

(wherein, R represents an alkyl group with a carbon number of 1-6, a cycloalkyl group with a carbon number of 3-6 or a phenyl group, and n represents an integer of 0-2).

[2] 1,3-Bis(4-hydroxyphenyl)-5-ethyladamantane represented by the following structural formula:

[Chemical Formula 2]

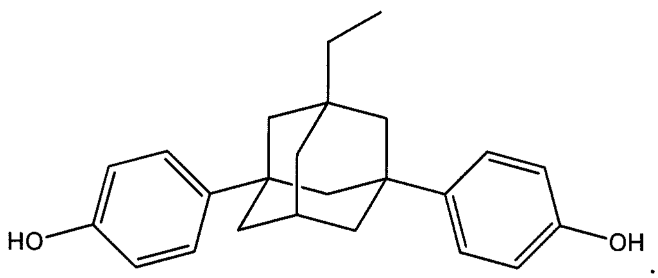

[3] 1,3-Bis(3-methyl-4-hydroxyphenyl)-5-ethyladamantane represented by the following structural formula:

[Chemical Formula 3]

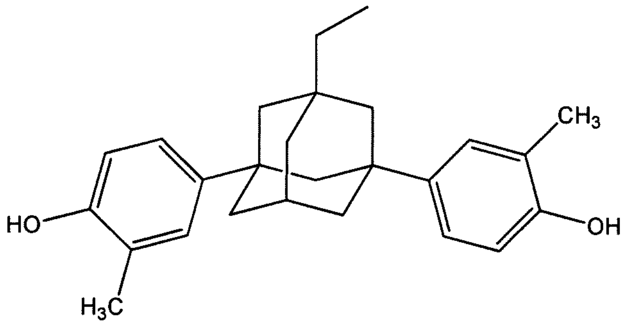

.

[4] A method for producing a 1,3-bis(hydroxyphenyl)-5-ethyladamantane compound represented by Formula (1) below, which comprises a step of reacting 1,3-dihydroxy-5-ethyladamantane represented by the following structural formula with phenol or a substituted phenol in the presence of an acid catalyst:

[Chemical Formula 4]

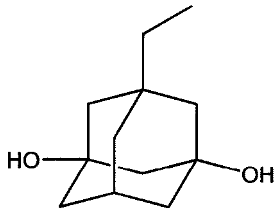

[Chemical Formula 5]

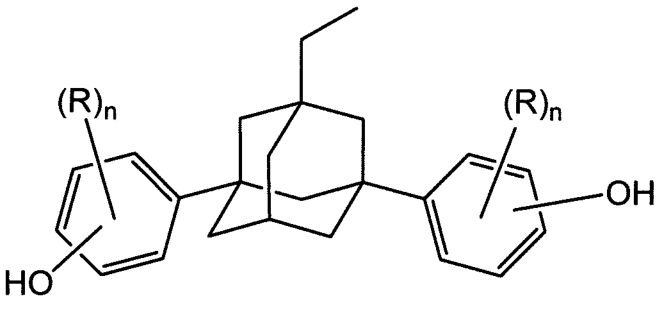

(1)

.

[5] A method for producing 1,3-bis(4-hydroxyphenyl)-5-ethyladamantane, which comprises a step of reacting 1,3-dihydroxy-5-ethyladamantane with phenol in the presence of an acid catalyst.

[6] A method for producing 1,3-bis(3-methyl-4-hydroxyphenyl)-5-ethyladamantane, which comprises a step of reacting 1,3-dihydroxy-5-ethyladamantane with o-cresol in the presence of an acid catalyst.

[7] An aromatic polycarbonate resin comprising a repeat unit represented by Formula (2) below and having a viscosity-average molecular weight of 1 x $10^4$ to 5 x $10^4$:

[Chemical Formula 6]

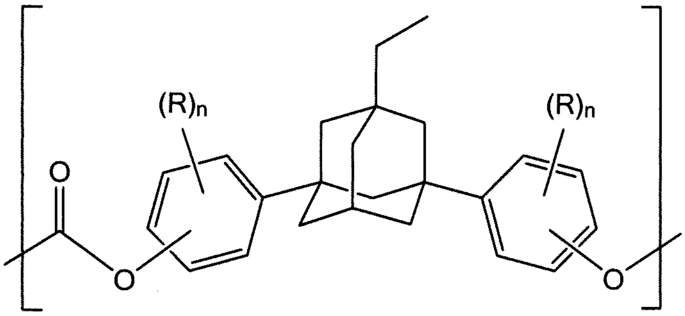

(2)

(wherein, R represents an alkyl group with a carbon number of 1-6, a cycloalkyl group with a carbon number of 3-6 or a phenyl group, and n represents an integer of 0-2).

[8] An aromatic polycarbonate resin comprising a repeat unit represented by Formula (3) below and having a viscosity-average molecular weight of 1 x $10^4$ to 5 x $10^4$:

[Chemical Formula 7]

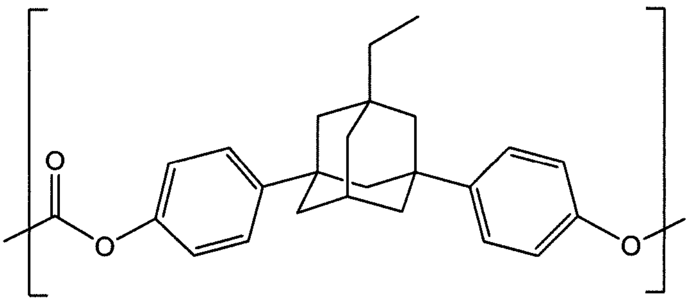

(3)

.

[9] An aromatic polycarbonate resin comprising a repeat unit represented by Formula (4) below and having a viscosity-average molecular weight of 1 x $10^4$ to 5 x $10^4$:

[Chemical Formula 8]

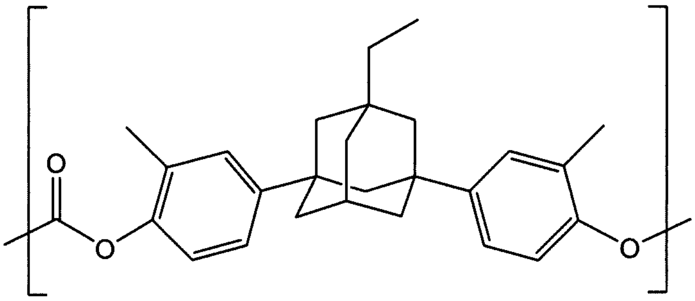

(4)

.

[10] The aromatic polycarbonate resin according to any one of [7] to [9], wherein the glass-transition temperature is 170-245°C.

[11] The method for producing an aromatic polycarbonate resin according to [7], which comprises a step of reacting 1,3-bis(hydroxyphenyl)-5-ethyladamantane compound represented by Formula (1) below with a carbonate-ester-forming compound:

[Chemical Formula 9]

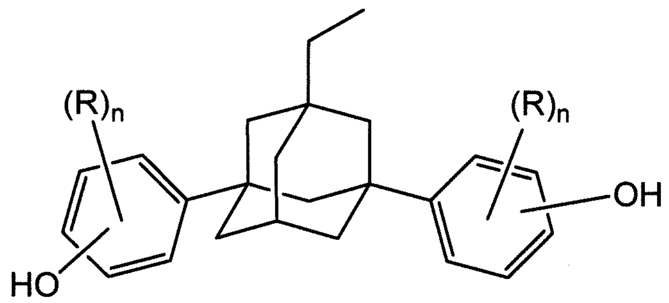

(1)

[12] The method for producing an aromatic polycarbonate resin according [8], which comprises a step of reacting 1,3-bis(4-hydroxyphenyl)-5-ethyladamantane with a carbonate-ester-forming compound.

[13] The method for producing an aromatic polycarbonate resin according to [9], which comprises a step of reacting 1,3-bis(3-methyl-4-hydroxyphenyl)-5-ethyladamantane with a carbonate-ester-forming compound.

EFFECT OF THE INVENTION

A 1,3-bis(hydroxyphenyl)-5-ethyladamantane compound represented by Formula (1) of the present invention is superior in optical properties and can favorably be used as a raw material for an aromatic polycarbonate resin that can realize both heat resistance and moldability.

BRIEF DESCRIPTION OF DRAWINGS

[Figure 1] Figure 1 shows a result from GC/MS analysis of the product obtained in Example 1.
[Figure 2] Figure 2 shows a result from $^{1}$H-NMR analysis of the product obtained in Example 1.
[Figure 3] Figure 3 shows assignments of $^{1}$H-NMR peaks of the product obtained in Example 1.
[Figure 4] Figure 4 shows a result from $^{13}$C-NMR analysis of the product obtained in Example 1.
[Figure 5] Figure 5 shows a result from DEPT135°-NMR analysis of the product obtained in Example 1.
[Figure 6] Figure 6 shows assignments of $^{13}$C-NMR peaks of the product obtained in Example 1.
[Figure 7] Figure 7 shows a result from the GC/MS analysis of the product obtained in Example 2.
[Figure 8] Figure 8 shows a result from the $^{1}$H-NMR analysis of the product obtained in Example 2.
[Figure 9] Figure 9 shows assignments of $^{1}$H-NMR peaks of the product obtained in Example 2.
[Figure 10] Figure 10 shows a result from the $^{13}$C-NMR analysis of the product obtained in Example 2.
[Figure 11] Figure 11 shows a result from DEPT135°-NMR analysis of the product obtained in Example 2.
[Figure 12] Figure 12 shows assignments of $^{13}$C-NMR peaks of the product obtained in Example 2.

MODES FOR CARRYING OUT THE INVENTION

Hereinafter, an embodiment for carrying out the present invention (hereinafter, simply referred to as "the present embodiment") will be described in detail. The following present embodiment is one example for illustrating the present invention, and there is no intention of limiting the present invention to the following description. The present invention can be carried out by appropriately modifying within the scope of the present invention.
A 1,3-bis(hydroxyphenyl)-5-ethyladamantane compound according to the present embodiment is represented by Formula (1) below:

[Chemical Formula 10]

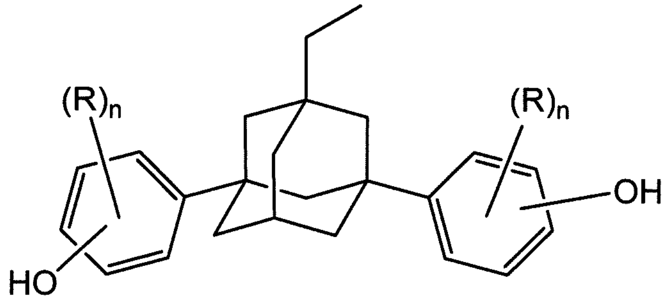 (1)

(wherein, R represents an alkyl group with a carbon number of 1-6, a cycloalkyl group with a carbon number of 3-6 or a phenyl group, and n represents an integer of 0-2).

In Formula (1), while -OH may be bonded at any of ortho-, meta- or para-position on the benzene ring, it is preferably bonded at para position (position 4). Similarly, while -R may be bonded at any position on the benzene ring, it is preferably bonded at position 3 or 5.

While an alkyl group with a carbon number of 1-6 represented by R in Formula (1) above is not particularly limited, examples include a methyl group, an ethyl group, an n-propyl group, an isopropyl group, an n-butyl group, an i-butyl group, a t-butyl group, an n-pentyl group and an n-hexyl group. Among them, a methyl group, an ethyl group and an n-propyl group are preferable, a methyl group and an ethyl group are more preferable and a methyl group is particularly preferable.

Examples of a cycloalkyl group with a carbon number of 3-6 represented by R in Formula (1) above include a cyclopropyl group, a cyclobutyl group, a cyclopentyl group and a cyclohexyl group, among which a cyclohexyl group is preferable.

Examples of the 1,3-bis(hydroxyphenyl)-5-ethyladamantane compound represented by Formula (1) above of the present embodiment include 1,3-bis(4-hydroxyphenyl)-5-ethyladamantane,
1,3-bis(3-methyl-4-hydroxyphenyl)-5-ethyladamantane,
1,3-bis(2-methyl-4-hydroxyphenyl)-5-ethyladamantane,
1,3-bis(3,5-dimethyl-4-hydroxyphenyl)-5-ethyladamantane,
1-(4-hydroxyphenyl)-3-(3-hydroxyphenyl)-5-ethyladamantane,
1-(4-hydroxyphenyl)-3-(2-hydroxyphenyl)-5-ethyladamantane,
1-(3-methyl-4-hydroxyphenyl)-3-(2-methyl-3-hydroxyphenyl)-5-ethyladamantane,
1-(3-methyl-4-hydroxyphenyl)-3-(4-methyl-3-hydroxyphenyl)-5-ethyladamantane,
1-(3-methyl-4-hydroxyphenyl)-3-(3-methyl-2-hydroxyphenyl)-5-ethyladamantane,
1,3-bis(3-ethyl-4-hydroxyphenyl)-5-ethyladamantane,
1,3-bis(2-ethyl-4-hydroxyphenyl)-5-ethyladamantane,
1,3-bis(3,5-diethyl-4-hydroxyphenyl)-5-ethyladamantane,
1-(3-ethyl-4-hydroxyphenyl)-3-(2-ethyl-3-hydroxyphenyl)-5-ethyladamantane,
1-(3-ethyl-4-hydroxyphenyl)-3-(4-ethyl-3-hydroxyphenyl)-5-ethyladamantane,
1-(3-ethyl-4-hydroxyphenyl)-3-(3-ethyl-2-hydroxyphenyl)-5-ethyladamantane and the like. Examples of a more preferable compound include
1,3-bis(4-hydroxyphenyl)-5-ethyladamantane,
1,3-bis(3-methyl-4-hydroxyphenyl)-5-ethyladamantane and
1,3-bis(3,5-dimethyl-4-hydroxyphenyl)-5-ethyladamantane. Examples of a still more preferable compound include 1,3-bis(4-hydroxyphenyl)-5-ethyladamantane and 1,3-bis(3-methyl-4-hydroxyphenyl)-5-ethyladamantane.

The 1,3-bis(hydroxyphenyl)-5-ethyladamantane compound represented by Formula (1) above of the present embodiment has a melting point of preferably 130 to 180°C, more preferably 135 to 175°C and particularly preferably 135°C to 170°C. Examples of a compound similar to the 1,3-bis(hydroxyphenyl)-5-ethyladamantane compound represented by Formula (1) above include 1,3-bis(4-hydroxyphenyl)-adamantane (melting point: 203 to 204°C) and 1,3-bis(4-hydroxyphenyl)-5,7-dimethyladamantane (melting point: 225°C). A melting point in a range of 130-180°C that is significantly lower than the melting points of these similar substances is beneficial in that handling in a molten state, for example, upon purification is easy.

The 1,3-bis(hydroxyphenyl)-5-ethyladamantane compound represented by Formula (1) above of the present embodiment can be used, for example, as a raw material for an epoxy resin, a photosensitive resin, a cyanate resin, a polyester resin, a polycarbonate resin or the like. By using this 1,3-bis(hydroxyphenyl)-5-ethyladamantane compound, a resin superior in terms of heat resistance, optical properties and mechanical strength properties can be produced.

A method for producing the 1,3-bis(hydroxyphenyl)-5-ethyladamantane compound of the present embodiment comprises a step of reacting 1,3-dihydroxy-5-ethyladamantane with phenol or a substituted phenol in the presence of an acid catalyst.

1,3-dihydroxy-5-ethyladamantane used as a raw material in the present embodiment can be synthesized by a known method such as: a method in which 1-ethyladamantane is subjected to oxygen oxidation in the presence of an imide compound and a vanadium compound; a method in which oxidization is carried out with hypochlorites in the presence of a ruthenium compound; a method in which 1-ethyladamantane is oxidized with chromic acid; and a method in which 1-ethyladamantane is dihalogenated and hydrolyzed.

According to the present embodiment, 1,3-dihydroxy-5-ethyladamantane synthesized by any of the above method or other method can be used.

According to the present embodiment, examples of phenol or a substituted phenol that is reacted with 1,3-dihydroxy-5-ethyladamantane include phenol, o-cresol, m-cresol, p-cresol, 2,6-xylenol, 2,3-xylenol, 2,4-xylenol, 2,5-xylenol, 3,4-xylenol and 3,5-xylenol.

In order to enhance the yield of a 1,3-bis(hydroxyphenyl)-5-ethyladamantane with respect to 1,3-dihydroxy-5-ethyladamantane, the phenol or the substituted phenol is preferably added at an excessive amount with respect to 1,3-dihydroxy-5-ethyladamantane, but too much amount only increases the amount of waste. The used amount of phenol or a substituted phenol is preferably 2 to 15 times and more preferably 6 to 10 times 1,3-dihydroxy-5-ethyladamantane in a molar ratio.

The acid catalyst used in the present embodiment may be any acid catalyst as long as it is a strong acid such as sulfuric acid, methanesulfonic acid, p-toluenesulfonic acid, trifluoromethane sulfonic acid, hydrochloric acid, hydrobromic acid, a strong acid cation exchange resin or the like but it is preferably a non-aqueous acid. Examples of a more preferable catalyst include concentrated sulfuric acid, methanesulfonic acid, p-toluenesulfonic acid, trifluoromethanesulfonic acid and a strong acid cation exchange resin.

The used amount of the above-described acid catalyst is usually preferably 10 to 300 mol%, more preferably 30 to 200 mol% and particularly preferably 50 to 150 mol% with respect to 1,3-dihydroxy-5-adamantane.

The reaction temperature according to the present embodiment is usually preferably 60 to 150°C, more preferably 80 to 130°C and particularly preferably 90 to 120°C.

The reaction method according to the present embodiment is not particularly limited and it may be, for example, a batch reaction method in which a raw material and an acid catalyst are placed in a reactor that is set at a predetermined reaction temperature for reaction.

A product obtained through the reaction according to the present embodiment may be subjected to neutralization and separation of the acid catalyst and then purified according to a conventional method such as distillation, extraction or column chromatography. By undergoing such purification, a highly pure novel 1,3-bis(hydroxyphenyl)-5-ethyladamantane compound represented by Formula (1) above can be obtained.

An aromatic polycarbonate resin of the present embodiment is a resin containing a repeat unit represented by Formula (2) below:

[Chemical Formula 11]

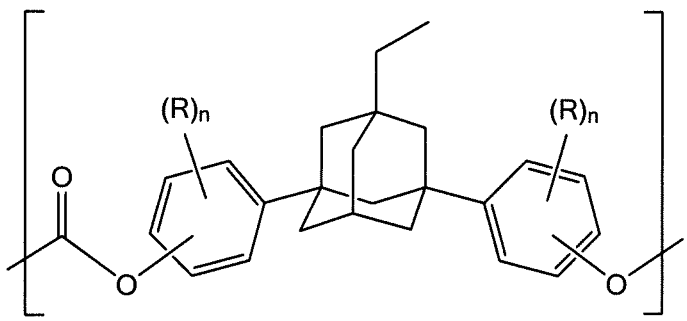

(2)

(wherein, R represents an alkyl group with a carbon number of 1-6, a cycloalkyl group with a carbon number of 3-6 or a phenyl group, and n represents an integer of 0-2).

In Formula (2) above, while -O- or -C(=O)-O- may be bonded at any of ortho-, meta- or para-position on the benzene ring, it is preferably bonded at para position (position 4). -OH is preferably bonded at the para position since that is favorable in terms of reactivity upon synthesizing a polycarbonate resin. Similarly, while -R may be bonded at any position on the benzene ring, it is preferably bonded at position 3 or 5 since that is favorable in terms of reactivity upon synthesizing a polycarbonate resin.

While an alkyl group with a carbon number of 1-6 represented by R in Formula (2) above is not particularly limited, examples include a methyl group, an ethyl group, an n-propyl group, an isopropyl group, an n-butyl group, an i-butyl group, a t-butyl group, an n-pentyl group and an n-hexyl group. Among them, a methyl group, an ethyl group and an n-propyl group are preferable, a methyl group and an ethyl group are more preferable and a methyl group is particularly preferable.

Examples of a cycloalkyl group with a carbon number of 3-6 represented by R in Formula (2) above include a cyclopropyl group, a cyclobutyl group, a cyclopentyl group and a cyclohexyl group, among which a cyclohexyl group is preferable.

A particularly preferable aromatic polycarbonate resin according to the present embodiment is a resin containing a repeat unit represented by Formula (3) or (4) below:

[Chemical Formula 12]

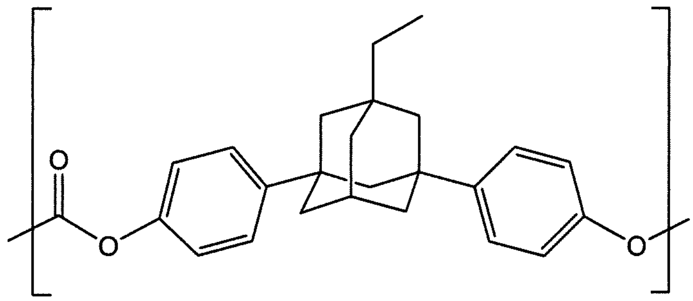

(3)

[Chemical Formula 13]

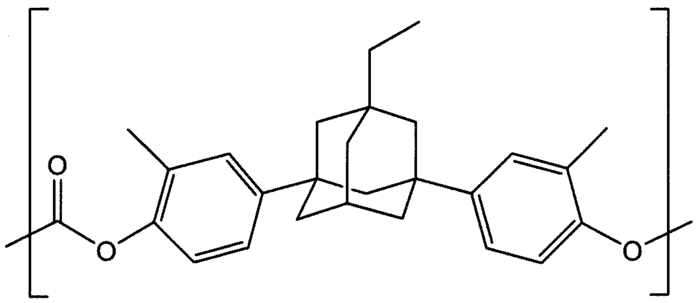

(4)

An aromatic polycarbonate resin of the present embodiment may contain a repeat unit other than the repeat unit represented by Formula (2) above as a copolymerization component.

The copolymerization component is not particularly limited as long as it is a component derived from an aromatic dihydroxy compound other than the aromatic dihydroxy compound represented by Formula (1) above, where examples include components derived from 2,2-bis(4-hydroxyphenyl)propane [=bisphenol A], bis(4-hydroxyphenyl)-p-diisopropylbenzene, 4,4'-dihydroxydiphenyl,
2,2-bis(4-hydroxy-3,5-dimethylphenyl)propane,
2,2-bis(4-hydroxy-3-methylphenyl)propane, 2,2-bis(4-hydroxy-3,5-diethylphenyl)propane, 2,2-bis(4-hydroxy-3,5-diphenylphenyl)propane,
2,2-bis(4-hydroxy-3,5-dibromophenyl)propane, 2,2-bis(4-hydroxyphenyl)pentane, 2,4'-dihydroxy-diphenylmethane, bis-(4-hydroxyphenyl)methane,
bis-(4-hydroxy-3-nitrophenyl)methane, 1,1-bis(4-hydroxyphenyl)ethane,
3,3-bis(4-hydroxyphenyl)pentane, 1,1-bis(4-hydroxyphenyl)cyclohexane [=bisphenol Z], bis(4-hydroxyphenyl)sulfone, 2,4'-dihydroxydiphenylsulfone, bis(4-hydroxyphenyl)sulfide,
4,4'-dihydroxydiphenylether, 4,4'-dihydroxy-3,3'-dimethyldiphenylether,
4,4'-dihydroxy-2,5-diethoxydiphenylether, 1-phenyl-1,1-bis(4-hydroxyphenyl)ethane,
1,1-bis(4-hydroxy-3-methylphenyl)cyclohexane,
1-phenyl-1,1-bis(4-hydroxy-3-methylphenyl)ethane,
bis(4-hydroxyphenyl)diphenylmethane, 9,9-bis(4-hydroxyphenyl)fluorene,
9,9-bis(4-hydroxy-3-methylphenyl)fluorene, 2,2-bis(4-hydroxyphenyl)hexafluoropropane,
1,3-bis(4-hydroxyphenyl)-adamantane, 1,3-bis(3-methyl-4-hydroxyphenyl)-adamantane,

and 1,3-bis(4-hydroxyphenyl)-5,7-dimethyladamantane and more preferable examples include components derived from 1,3-bis(4-hydroxyphenyl)-adamantane and
1,3-bis(4-hydroxyphenyl)-5,7-dimethyladamantane. These aromatic dihydroxy compounds may be used alone or two or more of them may be used as a mixture. In addition, as a part of a dihydroxy compound, a compound in which one or more tetraalkylphosphonium sulphonates are bonded to the above-described aromatic dihydroxy compound, a polymer or an oligomer having a siloxane structure with phenolic OH groups at both ends, or the like can be used in combination.

The viscosity-average molecular weight of an aromatic polycarbonate resin of the present embodiment is preferably 1 x $10^4$ to 5 x $10^4$, and more preferably 1.5 x $10^4$ to 4 x $10^4$. Within such a range, a good balance between fluidity and mechanical strength during molding can be kept more effectively.

The viscosity-average molecular weight (Mv) is calculated according to the following equation by measuring a 0.5 gram/deciliter dichloromethane solution of the aromatic polycarbonate resin with an Ubbelohde capillary viscometer at a temperature of 25°C, and determining the limiting viscosity [η] (deciliter/gram) at a Huggins constant of 0.45.

[Mathematical Formula 1]

$$\eta = 1.23 \times 10^{-4} \times Mv^{0.83}$$

The glass-transition temperature of an aromatic polycarbonate resin of the present embodiment is preferably 170-245°C, more preferably 175-240°C, and particularly preferably 180-230°C. The glass-transition temperature in a range of 170-245°C can result a resin having a sufficient heat resistance and good moldability that allows molding by various methods.

An aromatic polycarbonate resin of the present embodiment can be synthesized based on a known method including, for example, various synthesis methods such as an interfacial polymerization method and a transesterification method. Specifically, it is a linear or branched thermoplastic aromatic polycarbonate polymer or copolymer that is obtained by reacting an aromatic dihydroxy compound or an aromatic dihydroxy compound and a small amount of polyhydroxy compound with carboxyl chloride generally known as phosgene or a carboxyl compound such as diester carbonate as typified by dimethyl carbonate or diphenyl carbonate, carbon monoxide or carbon dioxide.

In order to obtain a branched aromatic polycarbonate resin, a polyhydroxy compound represented by phloroglucin, 4,6-dimethyl-2,4,6-tris(4-hydroxyphenyl)heptene-2, 4,6-dimethyl-2,4,6-tris(4-hydroxyphenyl)heptane,
2,6-dimethyl-2,4,6-tris(4-hydroxyphenyl)heptene-3,1,3,5-tris(4-hydroxyphenyl)benzene, 1,1,1-tris(4-hydroxyphenyl)ethane, or 3,3-bis(4-hydroxyaryl)oxindole (=isatin bisphenol), 5-chloroisatin bisphenol, 5,7-dichloroisatin bisphenol, 5-bromoisatin bisphenol or the like can be used as a part of the above-described aromatic dihydroxy compound, where the amount used is 0.01 to 10 mol% and preferably 0.1 to 3 mol%.

According to reaction based on an interfacial polymerization method, an aromatic dihydroxy compound and a molecular weight modifier (chain terminator), and if necessary an antioxidant for preventing oxidation of the aromatic dihydroxy compound, are used for reaction with phosgene in the presence of an organic solvent inactive to reaction and an aqueous alkaline solution while keeping pH usually at 10 or higher, then a polymerization catalyst such as a tertiary amine or a quaternary ammonium salt is added for interfacial polymerization, thereby obtaining an aromatic polycarbonate resin. Addition of the molecular weight modifier is not particularly limited as long as it is added during a period between the phosgenation and the start of the polymerization reaction. The reaction temperature is 0 to 35°C while the reaction time is several minutes to several hours.

Examples of the organic solvent inactive to reaction include chlorinated hydrocarbons such as dichloromethane, 1,2-dichloroethane, chloroform, monochlorobenzene and dichlorobenzene, and aromatic hydrocarbons such as benzene, toluene and xylene. As the molecular weight modifier or the chain terminator, a compound having a monovalent phenolic hydroxyl group can be used, specific examples being m-methylphenol, p-methylphenol, m-propylphenol, p-propylphenol, p-tert-butylphenol and p-long-chain alkyl-substituted phenol. Examples of the polymerization catalyst include tertiary amines such as trimethylamine, triethylamine, tributylamine, tripropylamine, trihexylamine, pyridine; and quaternary ammonium salts such as trimethylbenzyl ammonium chloride, tetramethyl ammonium chloride and triethylbenzyl ammonium chloride.

Reaction in the transesterification method is transesterification reaction between diester carbonate and an aromatic dihydroxy compound. Usually, a molecular weight and a terminal hydroxyl group quantity of a desirable aromatic polycarbonate resin are determined by adjusting the mixture ratio of diester carbonate and the aromatic dihydroxy compound or by adjusting the decompression degree upon reaction. The terminal hydroxyl group quantity has a major effect on heat stability, hydrolysis stability, color tone and the like of the aromatic polycarbonate resin, and it is preferably 1000 ppm or less and more preferably 700 ppm or less in order to achieve practical physical properties. Diester carbonate is generally used at an equimolar amount or more with respect to 1 mole of the aromatic dihydroxy compound, which is preferably 1.01 to 1.30 moles.

Examples of diester carbonate include dialkyl carbonate compounds such as dimethyl carbonate, diethyl carbonate and di-tert-butyl carbonate, diphenyl carbonate or substituted diphenyl carbonates such as di-p-tolyl carbonate, phenyl-p-tolyl carbonate, di-p-chlorophenyl carbonate. Among them, diphenyl carbonate and substituted diphenyl carbonates are preferable, and diphenyl carbonate is particularly preferable.

These diester carbonate compounds can be used alone or two or more of them can be used as a mixture.

When an aromatic polycarbonate resin is synthesized by a transesterification method, a transesterification catalyst is usually used. Although the transesterification catalyst is not particularly limited, an alkali metal compound and/or an alkaline-earth metal compound is primarily used, where a basic compound such as a basic boron compound, a basic phosphorous compound, a basic ammonium compound or an amine compound can supplementarily be used in combination. Transesterification reaction using such raw materials may be conducted, for example, as follows: the reaction is performed at a temperature of 100-320°C, and finally melt polycondensation reaction is conducted under a reduced pressure of 2.7 x $10^2$Pa (2 mmHg) or less while removing a by-product such as an aromatic hydroxy compound. According to the transesterification method, as a deactivating agent for the catalyst in the aromatic polycarbonate resin, i.e., a compound for neutralizing the catalyst, for example, a sulfur-containing acid compound or a derivative therefrom is preferably used, whose amount is 0.5 to 10 equivalent and preferably in a range of 1 to 5 equivalent relative to the alkali metal of the catalyst, and which is added to the aromatic polycarbonate resin usually in a range of 1 to 100 ppm and preferably in a range of 1 to 20 ppm.

To the aromatic polycarbonate resin of the present embodiment, various additives may be added without departing from the scope of the invention. Such additive may be, for example, at least one selected from the group consisting of a heat stabilizer, an antioxidant, a flame retardant, an ultraviolet absorber, mold-releasing agent and a colorant.

Additionally, as long as the various desirable physical properties are not seriously impaired, an antistatic agent, a fluorescent brightener, an antifog agent, a fluidity improving agent, a plasticizer, a dispersant, an antimicrobial agent and the like may also be added.

Since the aromatic polycarbonate resin of the present embodiment has superior optical properties, heat resistance and mechanical properties, it can favorably be used for the purposes of various lenses and a material for various optical apparatuses such as a liquid crystal panel.

Since the aromatic polycarbonate resin of the present embodiment is superior in moldability, a method for producing a molded article for these purposes is not particularly limited, and any molding technique generally employed for a polycarbonate resin composition can be employed. Examples of such technique include an injection molding technique, an ultrahigh-speed injection molding technique, an injection compression molding technique, a two-color molding technique, a hollow molding technique such as a gas assist molding, a molding technique that utilizes a thermally insulated die, a molding technique that utilizes a rapidly heated die, foam molding (including a supercritical fluid), insert molding, IMC (in-mold coating) technique, an extrusion molding technique, a sheet molding technique, a heat molding technique, a rotational molding technique, a lamination molding technique and a press molding technique.

## EXAMPLES

Hereinafter, the present invention will be described in more detail by way of examples below although the present invention should not be limited to these examples.

<Method of analysis>

(1) GC-FID analysis

Agilent capillary column DB-1 30m with an inner diameter of 0.53 mm and a film thickness of 1.5 μm was attached to Hewlett Packard gas chromatograph HP-6890 to conduct an analysis with a FID detector.

(2) GC/MS analysis

Agilent capillary column DB-1MS 30m with an inner diameter of 0.250 mm and a film thickness of 0.25μm was attached to Shimadzu gas chromatograph mass spectrometer GCMS-QP2010 Ultra to conduct an analysis.

(3) NMR analysis

A sample to be measured was dissolved in acetone-D6 to make a 10% solution, and measured using JEOL JNM-AL400 nuclear magnetic resonator.

(4) Melting point

METTLER TOLEDO fully automatic melting point measuring apparatus FP62 was used to measure the melting point. The melting point upon the temperature rising of 0.2°C/min. was measured.

(5) Measurement of viscosity-average molecular weight (Mv) of polycarbonate resin

A viscosity-average molecular weight (Mv) was calculated according to the following equation by measuring a 0.5 gram/deciliter dichloromethane solution of the aromatic polycarbonate resin with an Ubbelohde capillary viscometer at a temperature of 25°C, and determining the limiting viscosity [η] (deciliter/gram) at a Huggins constant of 0.45.

[Mathematical Formula 2]

$$\eta = 1.23 \times 10^{-4} \times Mv^{0.83}$$

(6) Measurement of glass-transition point of polycarbonate resin

Seiko Instruments DSC220 was used in a nitrogen gas flow environment of 50 ml/min to perform a sample pretreatment by heating/melting at 270°C and then a measurement at a temperature increase rate of 10°C/min.

<Product example> Synthesis of 1,3-dihydroxy-5-ethyladamantane

1,3-dihydroxy-5-ethyladamantane was synthesized according to the method described in Example VI of US Patent Publication No. 3383424.

Specifically, to a 5L separable flask, 1,890 g of an aqueous acetic acid solution (moisture concentration 15%) and 609 g of chromium trioxide as a chromic acid source were placed, to which 200 g of 1-ethyladamantane was added dropwise spending 30 minutes while stirring with a mechanical stirrer. During the addition, the solution produced heat and the solution temperature increased from room temperature to as high as 90°C. Thereafter, the solution temperature was maintained at 80 to 90°C to continue the reaction for 3.5 hours.

At the end of the reaction, the reaction product was cooled to room temperature, and the precipitated crystal was separated through a glass filter, thereby obtaining 221 g of crude crystal of 1,3-dihydroxy-5-ethyladamantane. The resulting crude crystal was purified through recrystallization with acetone to obtain 204g of 1,3-dihydroxy-5-ethyladamantane with a GC purity of 99.2%.

<Production of 1,3-bis(hydroxyphenyl)-5-ethyladamantane compound>

<Example 1>

Synthesis of 1,3-bis(4-hydroxyphenyl)-5-ethyladamantane

170 g (0.866 moles) of 1,3-dihydroxy-5-ethyladamantane and 652 g (6.93 moles) of phenol were placed into a 2L separable flask and the temperature was increased to 85°C while stirring with a mechanical stirrer. Once the temperature reached 85°C, 85 g (0.867 moles) of concentrated sulfuric acid was added dropwise spending 20 minutes. During the addition, the solution produced heat and the temperature of the solution increased as high as 95°C. Once the addition is completed, the solution temperature was adjusted to 90°C to continue the reaction for 5 hours. After 5 hours, the reaction solution was poured into a container with a 2L of ice water. Furthermore, a 24% aqueous sodium hydroxide solution was added until pH becomes 7 for neutralization.

To the resulting reaction product, 500 ml of ethyl acetate was added to repeat extraction with ethyl acetate for three times. The extracted ethyl acetate solution was washed with 500 ml of saturated saline, and magnesium sulfate was further added to the separated ethyl acetate solution phase.

Magnesium sulfate was removed from the ethyl acetate solution by filtration and ethyl acetate was concentrated with an evaporator. To 650 g of the concentrated solution, 1.5 L of hexane was added, stirred and left to stand so that the solution separated into two layers. The supernatant hexane layer was separated by decanting. Washing with 1.5 L of hexane was further repeated twice.

The washed solution was purified by column chromatography. A silica gel column was used to first develop with toluene. Development was carried out with toluene until phenol was no longer detected. Then, development was carried out with a toluene: ethyl acetate = 4: 1 mixed solvent. The toluene/ethyl acetate mixed solvent was concentrated

with an evaporator and further dried with a drier, thereby obtaining 172 g of an oily product. The ethanol solution of the product was subjected to GC-FID analysis. As a result of which, the peak area % other than the solvent was 99.51 %.

The melting point of the resulting product was measured to be 164 to 167°C.

<Identification of product of Example 1>

The result from the GC/MS analysis of the product obtained in Example 1 is shown in Figure 1. From the mass spectrum, the molecular weight of the product seemed to be 348.

The NMR measurement results of the product obtained in Example 1 are shown below.

$^1$H -NMR (400MHz) (ACETONE-D6) $\delta$: 8.07 (2H, s), 7.24 (4H, dt, J=9.3, 2.6Hz), 6.78 (4H, dt, J=9.3, 2.6Hz), 2.31-2.28 (1H, m), 1.86-1.79 (6H, m), 1.58 (4H, br s), 1.49 (2H, br s), 1.27 (2H, q, J=7.6Hz), 0.86 (3H, t, J=7.6Hz)

$^{13}$C-NMR (100MHz) (ACETONE-D6) $\delta$: 156.0, 142.5, 126.7, 115.6, 50.1, 48.0, 42.9, 40.9, 38.0, 37.0, 34.9, 31.0, 7.4

Figure 2 shows the $^1$H-NMR chart and Figure 3 shows the assignments of the $^1$H-NMR peaks. Figure 4 shows the $^{13}$C-NMR chart, Figure 5 shows the DEPT135°-NMR chart, and Figure 6 shows the assignments of the $^{13}$C-NMR peaks. From comprehensive assessment of these measurement results, the chief component of the product obtained in Example 1 was identified as 1,3-bis(4-hydroxyphenyl)-5-ethyladamantane.

Meanwhile, the melting points of 1,3-bis(4-hydroxyphenyl)adamantane and 1,3-bis(4-hydroxyphenyl)-5,7-dimethyladamantane, which were not comprised by a compound of the present invention represented by Formula (1), were 203 to 204°C and 225°C, respectively.

<Example 2>

Synthesis of 1,3-bis(3-methyl-4-hydroxyphenyl)-5-ethyladamantane

170 g (0.866 moles) of 1,3-dihydroxy-5-ethyladamantane and 750 g (6.94 moles) of o-cresol were placed in a 2L separable flask, and the temperature was increased to 65°C while stirring with a mechanical stirrer. Once the temperature reached 65°C, 85 g (0.867 moles) of concentrated sulfuric acid was added dropwise spending 30 minutes. During the addition, the solution produced heat and the temperature of the solution increased as high as 90°C. Once the addition was completed, the solution temperature was adjusted to 93°C to continue the reaction for 7 hours. After 7 hours, 600 ml of a toluene: ethyl acetate = 1: 1 mixed solvent was further added for dilution and a 24% aqueous sodium hydroxide solution was added until the solution was neutralized.

To the resulting reaction product, 500 ml of ethyl acetate was added to repeat extraction with ethyl acetate for three times. The extracted ethyl acetate solution was washed with 500 ml of saturated saline, and magnesium sulfate was further added to the separated ethyl acetate solution phase.

Magnesium sulfate was removed from the ethyl acetate solution by filtration and ethyl acetate was concentrated with an evaporator. To 550 g of the concentrated solution, 1 L of hexane was added, stirred and left to stand so that the solution separated into two layers. The supernatant hexane layer was separated by decanting. Washing with 1 L of hexane was further repeated twice.

The washed solution was purified by column chromatography. A silica gel column was used to first develop with toluene. Development was carried out with toluene until o-cresol was no longer detected. Then, development was carried out with a toluene: ethyl acetate = 4: 1 mixed solvent. The toluene/ethyl acetate mixed solvent was concentrated with an evaporator and further dried with a drier, thereby obtaining 103 g of an oily product. The ethanol solution of the product was subjected to GC-FID analysis. As a result of which, the peak area % other than the solvent was 91.60%.

The melting point of the resulting product was measured to be 146 to 149°C.

<Identification of product of Example 2>

The result from the GC/MS analysis of the product obtained in Example 2 is shown in Figure 7. From the mass spectrum, the molecular weight of the product seemed to be 376.

The NMR measurement results of the product obtained in Example 2 are shown below.

$^1$H-NMR (400MHz) (ACETONE-D6) $\delta$: 7.89 (2H, s), 7.15 (2H, d, J=2.2Hz), 7.04 (2H, dd, J=8.3, 2.2Hz), 6.74 (2H, d, J=8.3Hz), 2.29-2.28 (1H, m), 2.19 (6H, s), 1.86-1.78 (6H, m), 1.57 (4H, br s), 1.48 (2H, br s), 1.26 (2H, q, J=7.6Hz), 0.86 (3H, t, J=7.6Hz)

$^{13}$C-NMR (100MHz) (ACETONE-D6) $\delta$: 153.9, 142.5, 128.1,124.1,123.8, 115.0, 50.2, 48.1, 42.9, 40.9, 37.9, 37.0, 34.9, 31.0, 16.5, 7.4

Figure 8 shows the $^1$H-NMR chart and Figure 9 shows the assignments of the $^1$H-NMR peaks. Figure 10 shows the $^{13}$C-NMR chart, Figure 11 shows the DEPT135°-NMR chart, and Figure 12 shows the assignments of the $^{13}$C-NMR

peaks. From comprehensive assessment of these measurement results, the chief component of the product obtained in Example 2 was identified as
1,3-bis(3-methyl-4-hydroxyphenyl)-5-ethyladamantane.

<Production of aromatic polycarbonate resin>

<Example 3>

To 600ml of an 4.5 w/w% aqueous sodium hydroxide, 51.6g of 1,3-bis(4-hydroxyphenyl)-5-ethyladamantane produced in Example 1 and 0.3g of hydrosulfite were added and dissolved. To this, 300 ml of dichloromethane was added, into which 23.5 g of phosgene was blown spending 20 minutes while stirring and keeping the solution temperature to stay in a range of 15°C to 25°C.

After blowing the phosgene, 100ml of dichloromethane and a solution of 0.402g of para-tertiary-butylphenol dissolved in 50ml of dichloromethane were added and vigorously stirred for emulsification. One ml of triethylamine was added as a polymerization catalyst to allow the resultant to polymerize for about 40 minutes.

The polymerized solution was separated into a water phase and an organic phase. The organic phase was neutralized with a phosphoric acid, and washing with pure water was repeated until pH became neutral. The organic solvent was evaporated and distilled away from this purified polycarbonate resin solution, thereby obtaining polycarbonate resin powder.

The resulting polycarbonate resin powder had a viscosity-average molecular weight of 35,500 and a glass-transition point of 230°C.

<Example 4>

Polycarbonate resin powder was obtained in the same manner as in Example 3 except that 55.8g of 1,3-bis(3-methyl-4-hydroxyphenyl)-5-ethyladamantane produced in Example 2 was used instead of 51.6g of 1,3-bis(4-hydroxyphenyl)-5-ethyladamantane.

The resulting polycarbonate resin powder had a viscosity-average molecular weight of 24,500 and a glass-transition point of 183°C.

<Comparative Example 1>

Polycarbonate resin powder was obtained in the same manner as in Example 3 except that 51.6g of 1,3-bis(4-hydroxyphenyl)-5,7-dimethyladamantane was used instead of 51.6g of 1,3-bis(4-hydroxyphenyl)-5-ethyladamantane.

The resulting polycarbonate resin powder had a viscosity-average molecular weight of 23,700 and a glass-transition point of 252°C. A glass-transition point as high as 252°C causes a problem of poor molding processability.

INDUSTRIAL APPLICABILITY

1,3-bis(hydroxyphenyl)-5-ethyladamantane compound represented by Formula (1) above of the present invention may be used as a raw material, for example, for an epoxy resin, a photosensitive resin, a cyanate resin, a polyester resin, a polycarbonate resin or the like. Since an aromatic polycarbonate resin using this
1,3-bis(hydroxyphenyl)-5-ethyladamantane compound is superior in terms of heat resistance, optical properties and mechanical strength properties, it has great industrial significance.

## Claims

**1.** A 1,3-bis(hydroxyphenyl)-5-ethyladamantane compound represented by Formula (1) below:

[Chemical Formula 1]

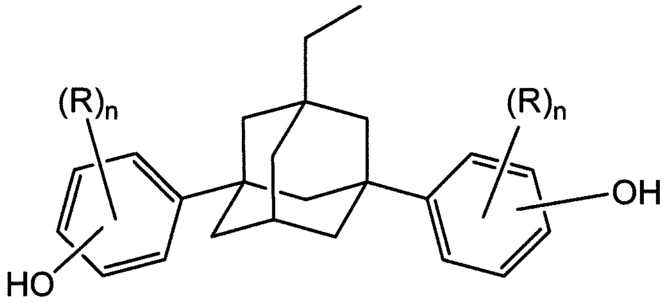

(1)

(wherein, R represents an alkyl group with a carbon number of 1-6, a cycloalkyl group with a carbon number of 3-6 or a phenyl group, and n represents an integer of 0-2).

**2.** 1,3-Bis(4-hydroxyphenyl)-5-ethyladamantane represented by the following structural formula:

[Chemical Formula 2]

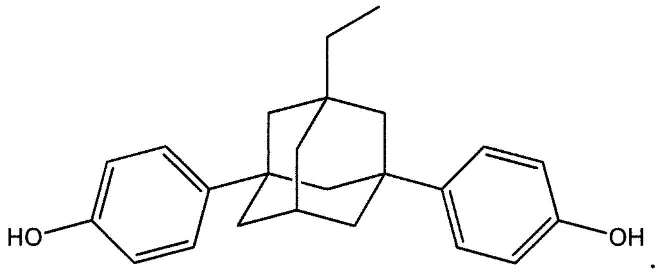

**3.** 1,3-Bis(3-methyl-4-hydroxyphenyl)-5-ethyladamantane represented by the following structural formula:

[Chemical Formula 3]

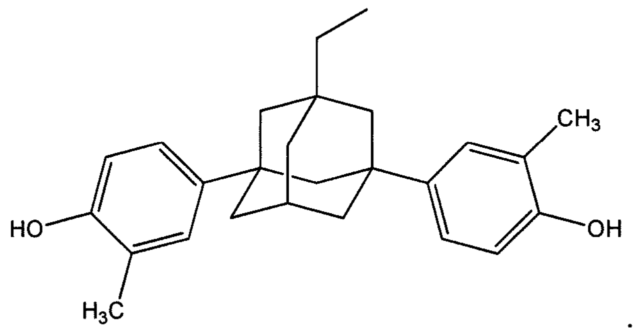

**4.** A method for producing a 1,3-bis(hydroxyphenyl)-5-ethyladamantane compound represented by Formula (1) below, which comprises a step of reacting 1,3-dihydroxy-5-ethyladamantane represented by the following structural formula with phenol or a substituted phenol in the presence of an acid catalyst:

[Chemical Formula 4]

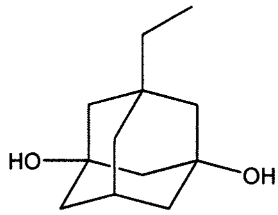

[Chemical Formula 5]

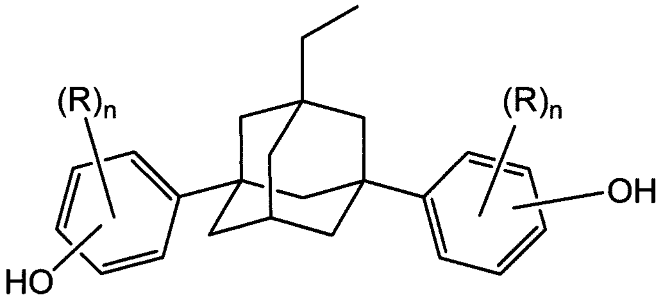

(1)

.

5. A method for producing 1,3-bis(4-hydroxyphenyl)-5-ethyladamantane, which comprises a step of reacting 1,3-dihydroxy-5-ethyladamantane with phenol in the presence of an acid catalyst.

6. A method for producing 1,3-bis(3-methyl-4-hydroxyphenyl)-5-ethyladamantane, which comprises a step of reacting 1,3-dihydroxy-5-ethyladamantane with o-cresol in the presence of an acid catalyst.

7. An aromatic polycarbonate resin comprising a repeat unit represented by Formula (2) below and having a viscosity-average molecular weight of 1 x $10^4$ to 5 x $10^4$:

[Chemical Formula 6]

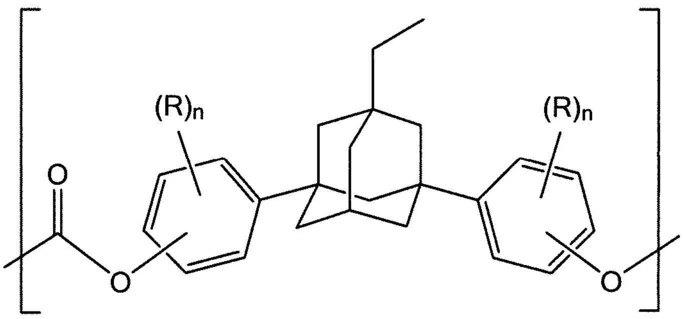

(2)

(wherein, R represents an alkyl group with a carbon number of 1-6, a cycloalkyl group with a carbon number of 3-6 or a phenyl group, and n represents an integer of 0-2).

8. An aromatic polycarbonate resin comprising a repeat unit represented by Formula (3) below and having a viscosity-average molecular weight of 1 x $10^4$ to 5 x $10^4$:

[Chemical Formula 7]

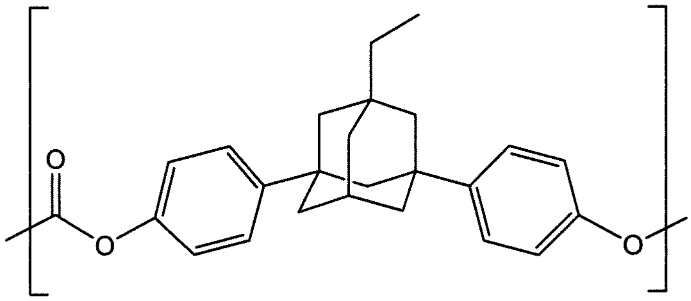

(3)

.

9. An aromatic polycarbonate resin comprising a repeat unit represented by Formula (4) below and having a viscosity-average molecular weight of 1 x $10^4$ to 5 x $10^4$:

[Chemical Formula 8]

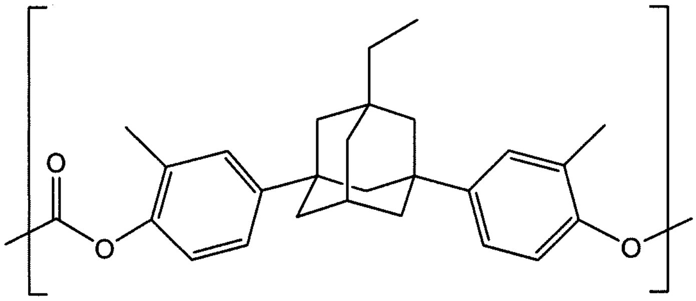

(4)

.

10. The aromatic polycarbonate resin according to any one of Claims 7 to 9, wherein the glass-transition temperature is 170 to 245°C.

11. The method for producing an aromatic polycarbonate resin according to Claim 7, which comprises a step of reacting 1,3-bis(hydroxyphenyl)-5-ethyladamantane compound represented by Formula (1) below with a carbonate-ester-forming compound:

[Chemical Formula 9]

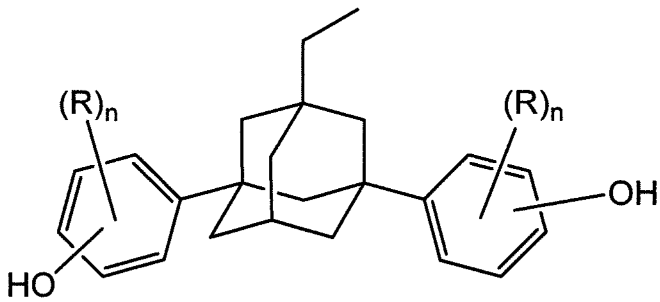

(1)

.

12. The method for producing an aromatic polycarbonate resin according Claim 8, which comprises a step of reacting 1,3-bis(4-hydroxyphenyl)-5-ethyladamantane with a carbonate-ester-forming compound.

13. The method for producing an aromatic polycarbonate resin according to Claim 9, which comprises a step of reacting 1,3-bis(3-methyl-4-hydroxyphenyl)-5-ethyladamantane with a carbonate-ester-forming compound.

[Figure 1]

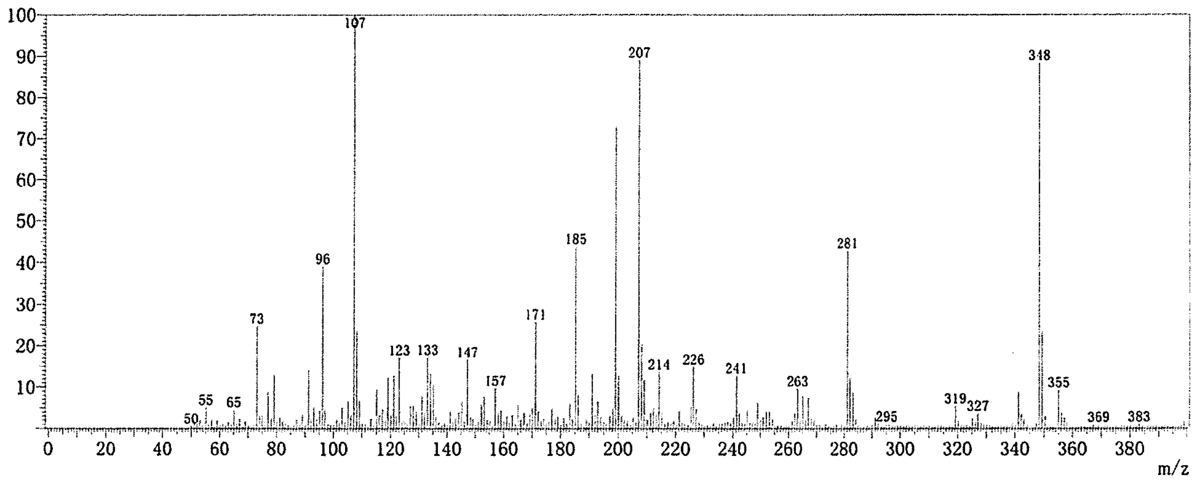
100
90
80
70
60
50
40
30
20
10
107
207
348
185
96
281
73
171
123
133
147
157
214
226
241
263
355
55
65
50
295
319
327
369
383
0
20
40
60
80
100
120
140
160
180
200
220
240
260
280
300
320
340
360
380
m/z

[Figure 2]

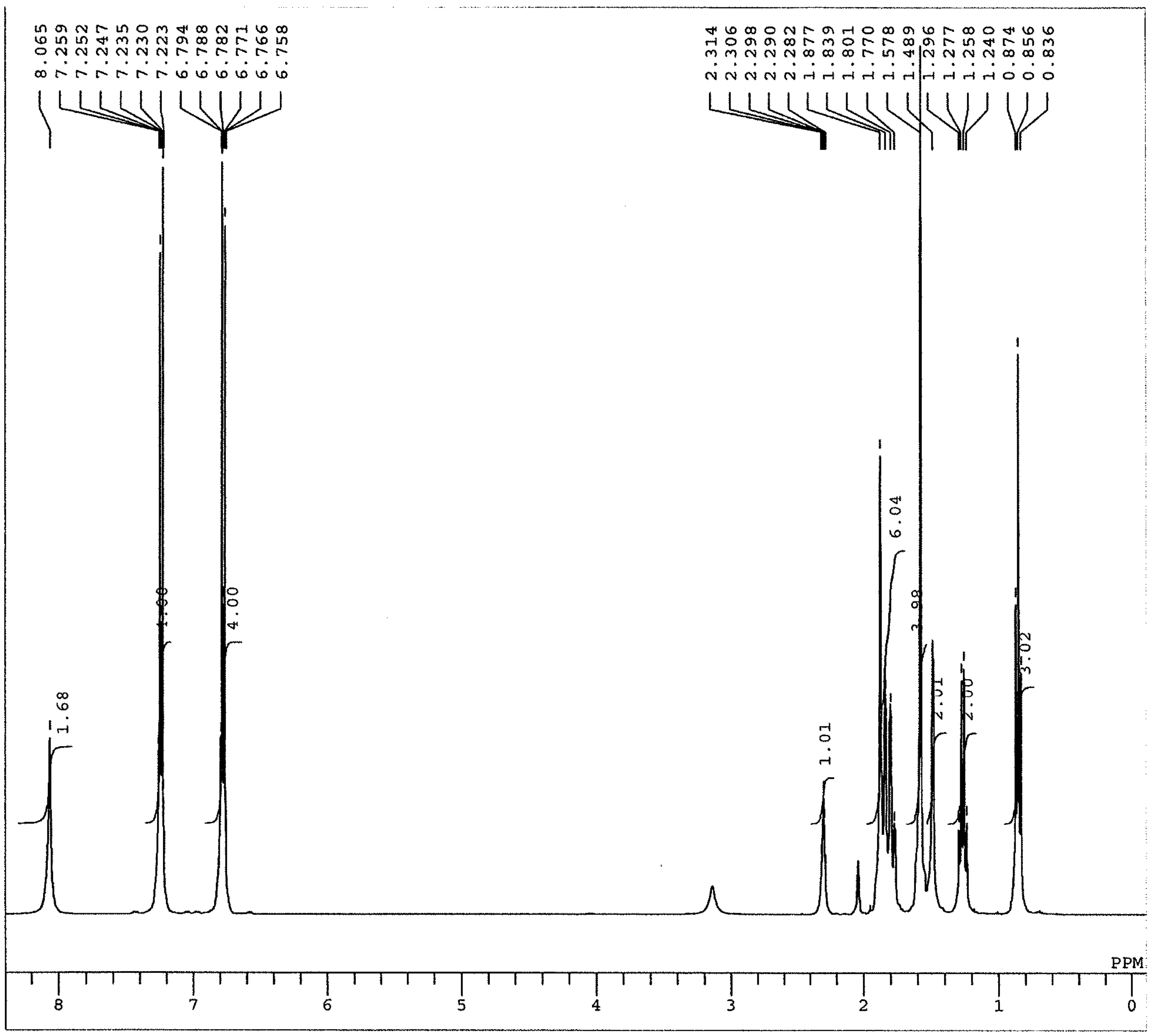


[Figure 3]

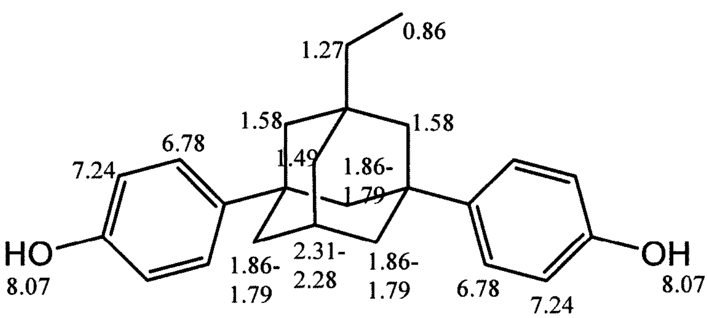

[Figure 4]

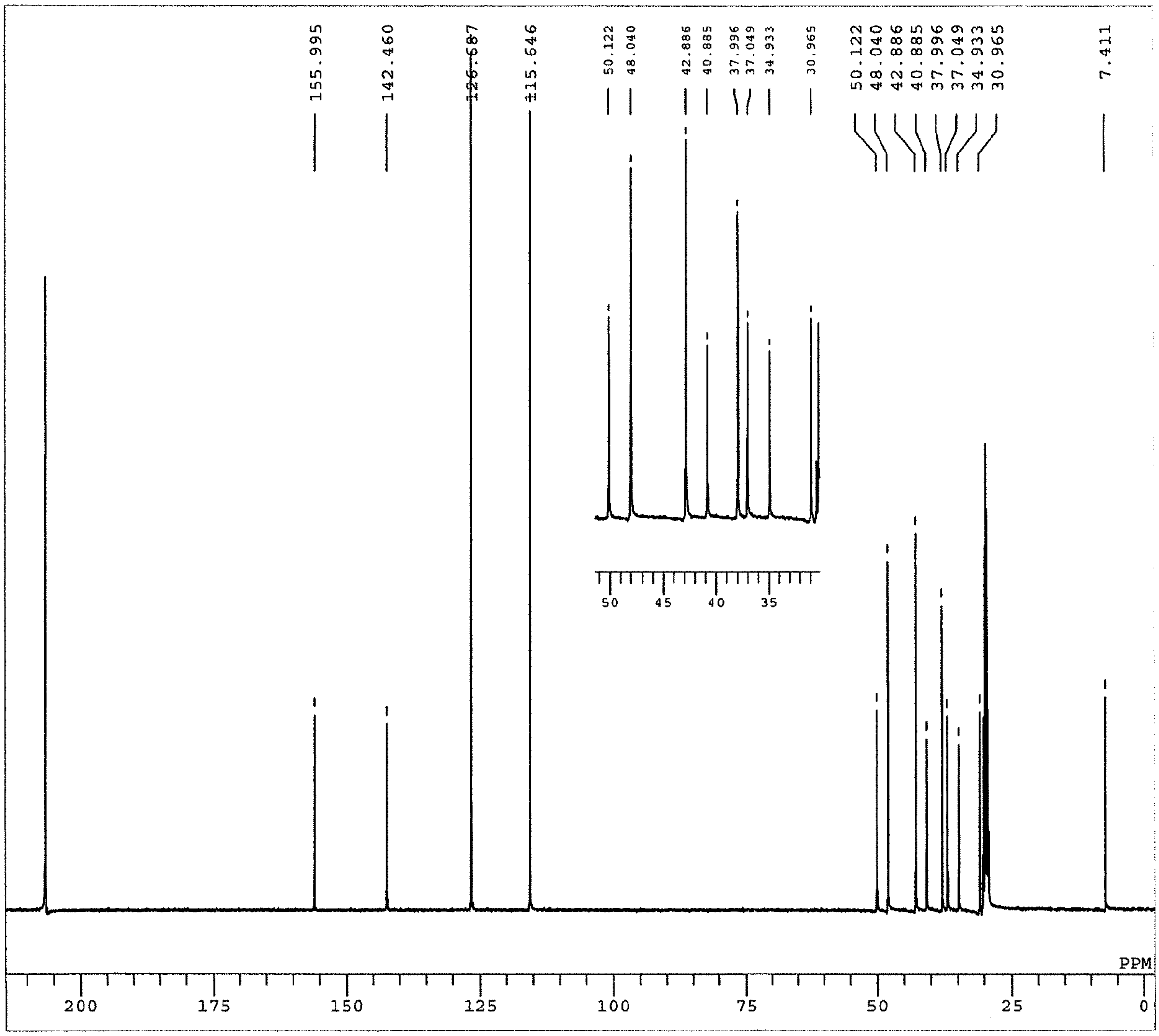
155.995
142.460
126.687
115.646
50.122
48.040
42.886
40.885
37.996
37.049
34.933
30.965
50.122
48.040
42.886
40.885
37.996
37.049
34.933
30.965
7.411
50
45
40
35
PPM
200
175
150
125
100
75
50
25
0

[Figure 5]

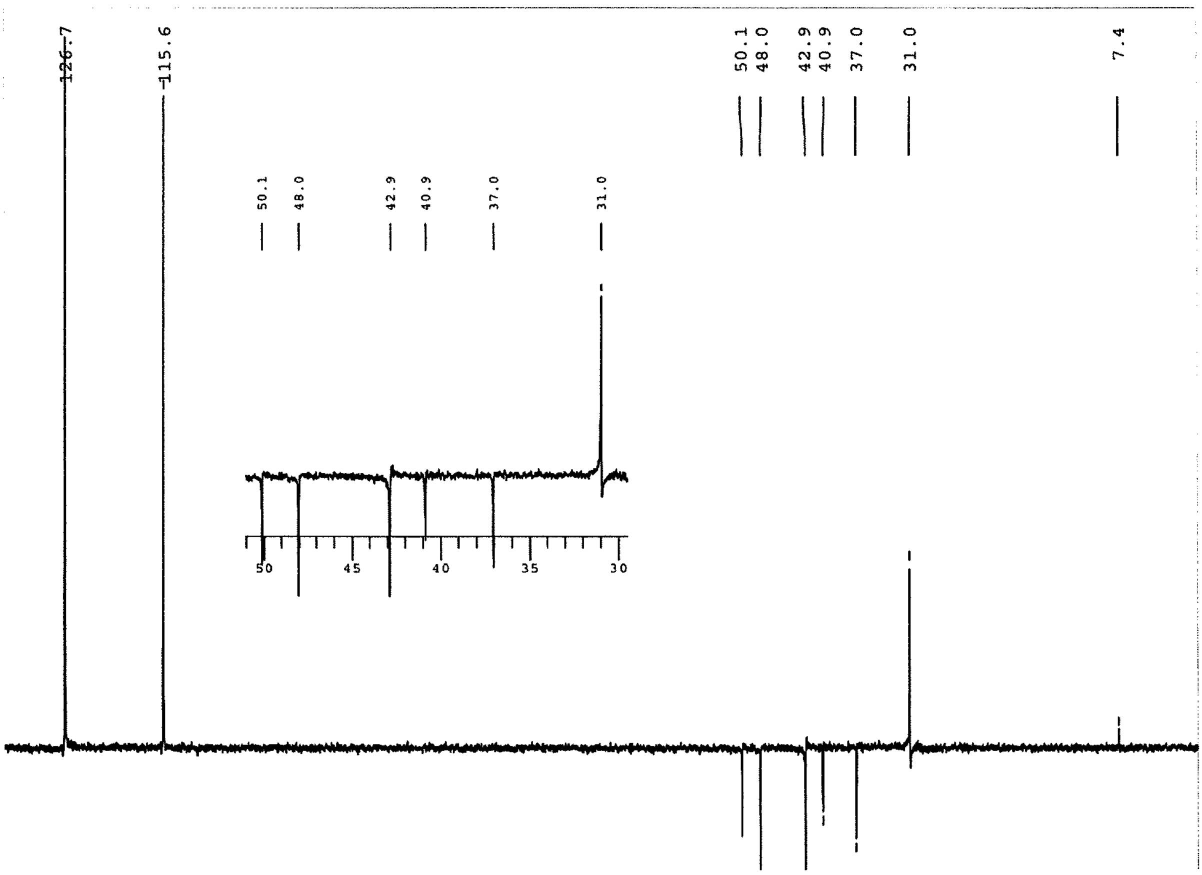


[Figure 6]

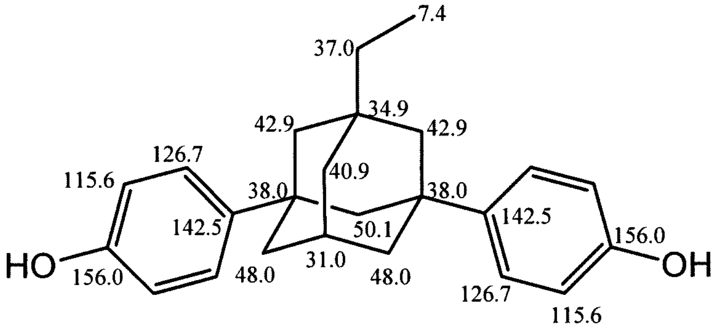

[Figure 7]

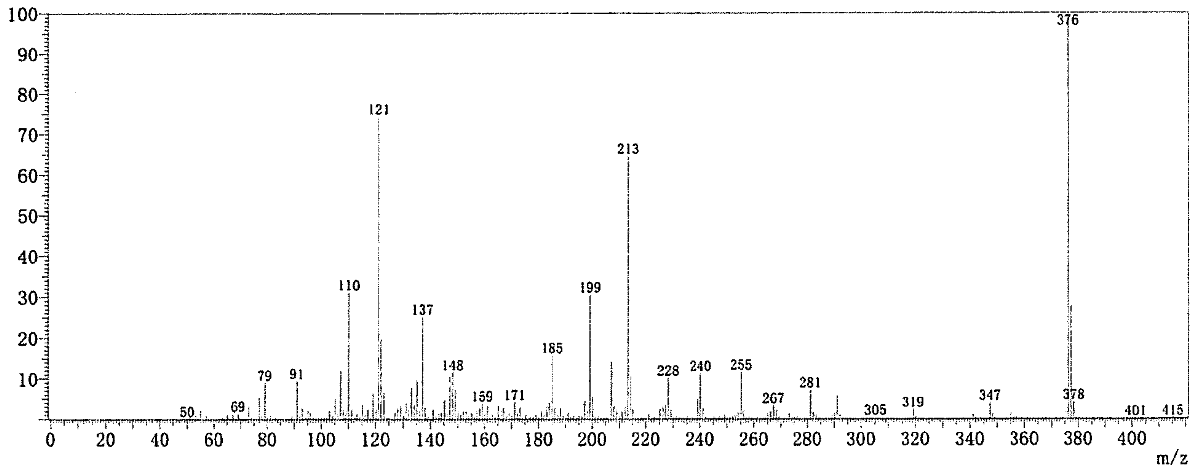
100
90
80
70
60
50
40
30
20
10
376
121
213
110
199
137
185
148
79
91
228
240
255
281
159
171
267
50
69
305
319
347
378
401
415
0
20
40
60
80
100
120
140
160
180
200
220
240
260
280
300
320
340
360
380
400
m/z

[Figure 8]

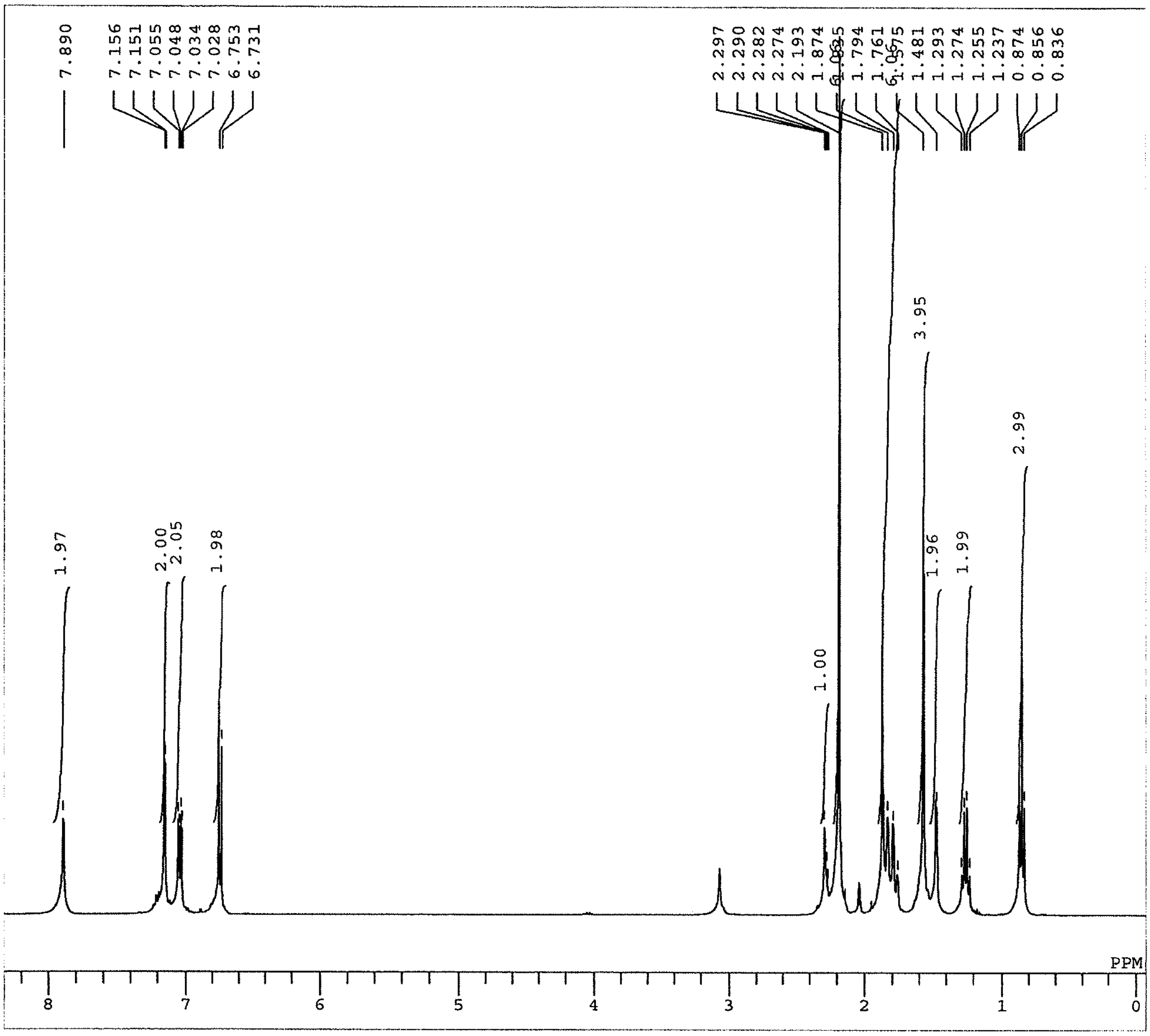


[Figure 9]

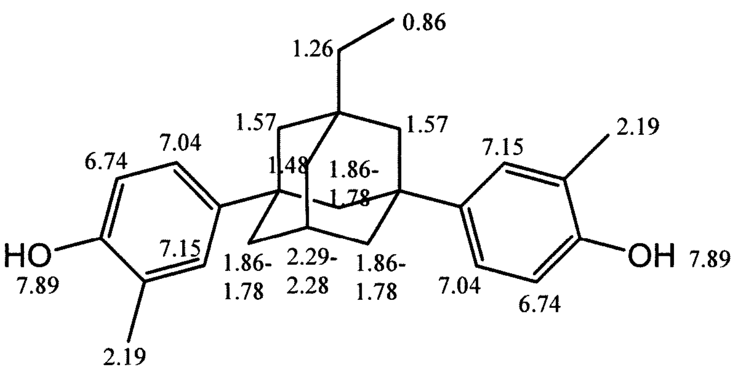

[Figure 10]

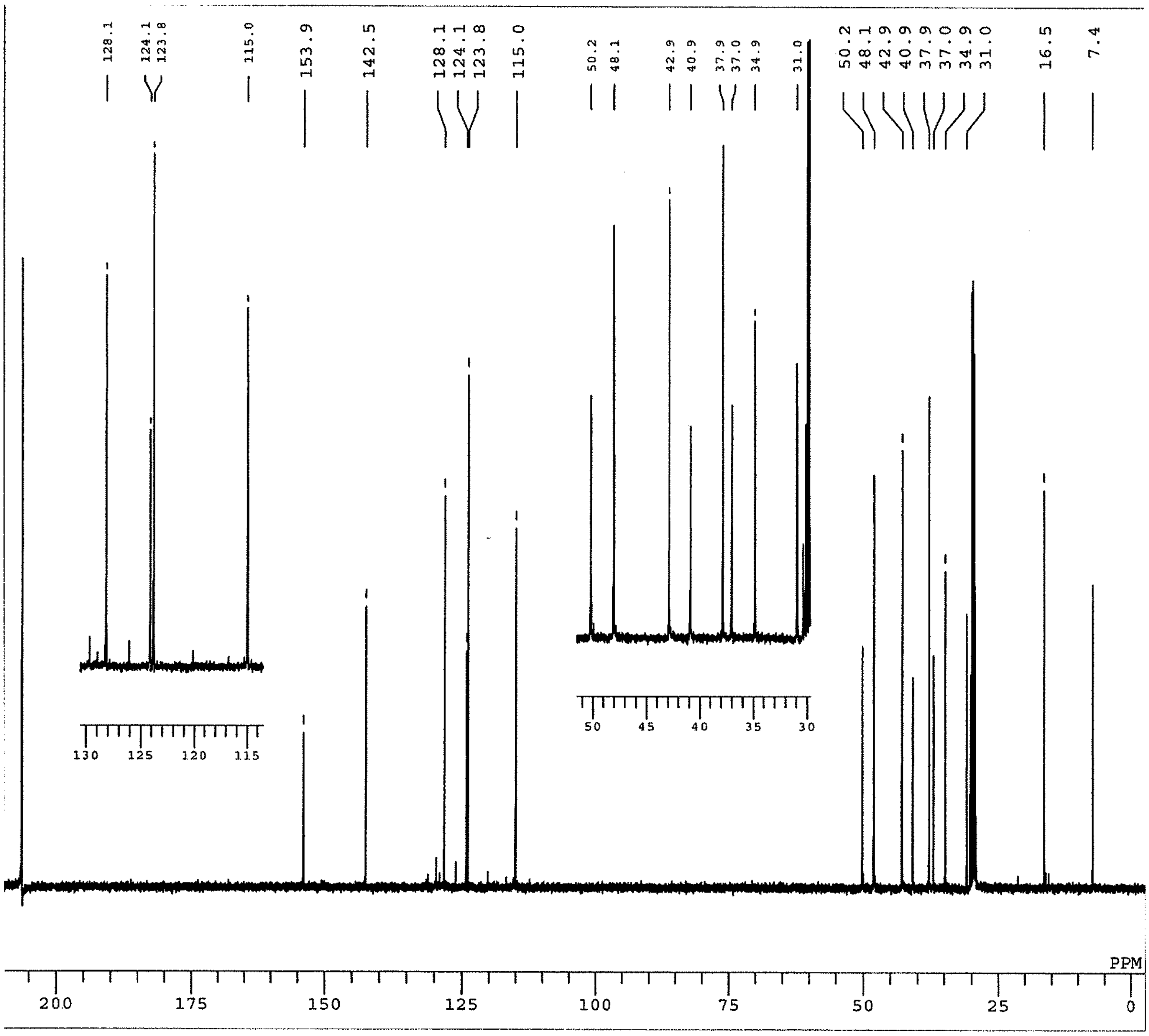
128.1
124.1
123.8
115.0
130 125 120 115
153.9
142.5
128.1
124.1
123.8
115.0
50.2
48.1
42.9
40.9
37.9
37.0
34.9
31.0
50 45 40 35 30
50.2
48.1
42.9
40.9
37.9
37.0
34.9
31.0
16.5
7.4
PPM
200 175 150 125 100 75 50 25 0

[Figure 11]

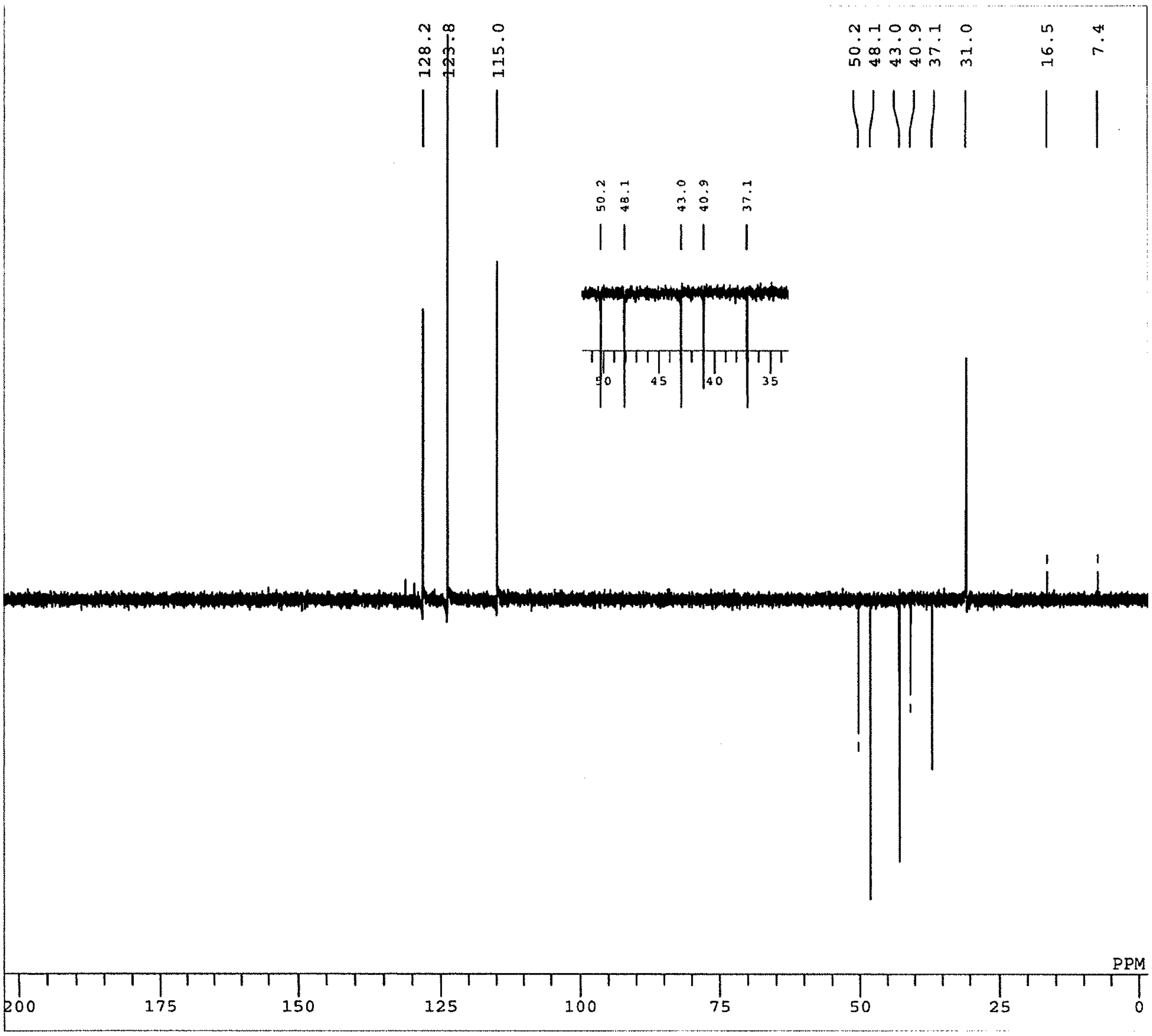


[Figure 12]

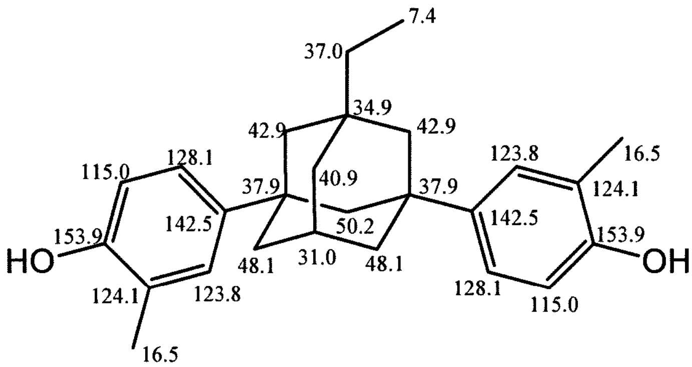

**INTERNATIONAL SEARCH REPORT**

International application No.
PCT/JP2014/078087

A. CLASSIFICATION OF SUBJECT MATTER
*C07C39/17*(2006.01)i, *B01J27/053*(2006.01)i, *C07C37/16*(2006.01)i, *C08G64/06*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C39/17, B01J27/053, C07C37/16, C08G64/06, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | US 3594427 A (SUN OIL CO.), 20 July 1971 (20.07.1971), column 2, line 20 to column 3, line 22; examples (Family: none) | 1-2<br>4-5<br>3,6-13 |
| Y<br>A | JP 2000-95720 A (Daicel Chemical Industries, Ltd.), 04 April 2000 (04.04.2000), claims; examples (Family: none) | 4-5<br>1-3,6-13 |
| Y<br>A | JP 2003-306460 A (Honshu Chemical Industry Co., Ltd.), 28 October 2003 (28.10.2003), claims; examples & US 2003/0187307 A1 & EP 1336597 A1 | 4-5<br>1-3,6-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 January 2015 (15.01.15) | 27 January 2015 (27.01.15) |

| Name and mailing address of the ISA/ | Authorized officer |
|---|---|
| Japan Patent Office | |
| Facsimile No. | Telephone No. |

Form PCT/ISA/210 (second sheet) (July 2009)

**INTERNATIONAL SEARCH REPORT**

International application No.
PCT/JP2014/078087

C (Continuation). DOCUMENTS CONSIDERED TO BE RELEVANT

| Category* | Citation of document, with indication, where appropriate, of the relevant passages | Relevant to claim No. |
|---|---|---|
| A | US 3383424 A (SUN OIL CO.), 14 May 1968 (14.05.1968), claims & GB 1078220 A & DE 1493134 A1 & FR 1461287 A & BE 674394 A & CH 467227 A & NL 6516807 A | 1-13 |
| A | JP 5-78467 A (Teijin Chemicals Ltd.), 30 March 1993 (30.03.1993), claims; examples (Family: none) | 1-13 |
| A | JP 2003-212987 A (Idemitsu Kosan Co., Ltd.), 30 July 2003 (30.07.2003), claims; examples & US 2005/0143553 A1 & US 2006/0270823 A1 & EP 1469026 A1 & WO 2003/062300 A1 & CN 1622965 A & KR 10-2004-0083431 A & KR 10-2008-0108625 A | 1-13 |
| A | JP 50-22099 A (Etat Francais), 08 March 1975 (08.03.1975), claims; examples & US 3897390 A & GB 1425286 A & DE 2424765 A & FR 2230679 A1 & NL 7405378 A | 1-13 |
| A | JP 10-17664 A (Teijin Chemicals Ltd.), 20 January 1998 (20.01.1998), claims; examples (Family: none) | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3594427 A
- JP 2000095720 A
- JP 2003306460 A
- US 3383424 A
- JP H0578467 A